# EUROPÄISCHE PATENTSCHRIFT

(11) **EP 0 744 394 B1**
(45) Veröffentlichungstag und Bekanntmachung des Hinweises auf die Patenterteilung: **09.12.1998**
(21) Anmeldenummer: 96107501.7
(22) Anmeldetag: 10.05.1996
(51) Int. Cl.: C07C 69/653, C08F 20/24, C08F 220/24

(54) **Fluoralkenylgruppen enthaltende (Meth-)Acrylate, deren Herstellung und Verwendung**
Perfluoroalkenyl-group-containing (meth)acrylates, their preparation and their use
(Méth)acrylates contenant des groupes perfluoroalkénoyl, leur préparation ainsique leur utilisation

(30) Priorität: 23.05.1995 DE 19518865
(43) Veröffentlichungstag der Anmeldung: 27.11.1996
(73) Patentinhaber: BAYER AG, 51368 Leverkusen (DE)
(72) Erfinder: Lui, Norbert, Dr., 51061 Köln (DE); Podszun, Wolfgang, Dr., 51061 Köln (DE)

(56) Entgegenhaltungen:
- EP-A- 0 234 724
- DE-A- 3 421 511

## Beschreibung

Die vorliegende Erfindung betrifft neue Fluoralkenylgruppen enthaltende (Meth-)-Acrylate, ein Verfahren zu deren Herstellung aus einem (Meth-)Acrylatgruppen enthaltenden Alkohol und einem Fluoralkylgruppen enthaltenden Olefin und deren Verwendung zur Herstellung von Beschichtungen mit hydrophoben und schmutzabweisenden Eigenschaften.

Polymerisierbare Perfluoralkylgruppen enthaltende Verbindungen sind bekannt (siehe z.B. DE-A 3 421 511). Zu ihrer Herstellung wird Perfluoroctancarbonsäurechlorid mit Pentaerythrittriacrylat umgesetzt. Nachteilig ist die geringe Stabilität der Esterbindung gegenüber Hydrolyse.

Es wurden nun Fluoralkenylgruppen enthaltende (Meth-)Acrylate der Formel (I) gefunden 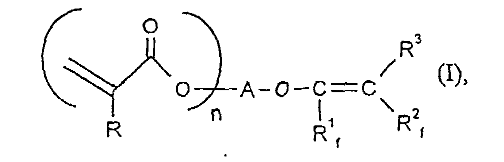 in der
- R: für Wasserstoff oder Methyl,
- n: für eine ganze Zahl von 2 bis 6,
- A: für einen (n+1)-wertigen, gegebenenfalls bis zu 10 Sauerstoffatome enthaltenden Kohlenwasserstoffrest mit 3 bis 30 C-Atomen und
- R¹_{f} und R²_{f}: unabhängig voneinander jeweils für einen Perfluoralkylrest mit 1 bis 20 C-Atomen stehen oder
- R¹_{f} und R²_{f}: gemeinsam eine 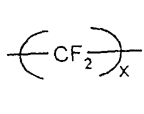 -Brücke bilden, wobei
- x: für eine ganze Zahl von 1 bis 8 steht und
- R³: für Wasserstoff, Fluor, Chlor oder einen Perfluoralkylrest mit 1 bis 20 C-Atomen steht.

In Formel (I) steht n vorzugsweise für eine ganze Zahl von 2 bis 5.

Bevorzugte Reste A enthalten 3 bis 12 C-Atome und gegebenenfalls bis zu 6 OH-Gruppen und/oder gegebenenfalls bis zu 8 Etherbrücken. Besonders bevorzugte Reste A sind: 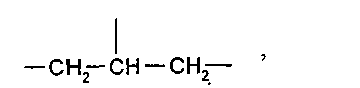 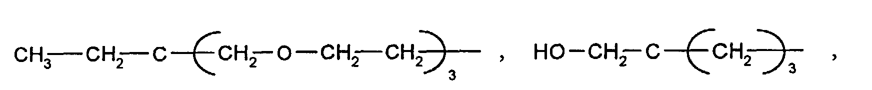 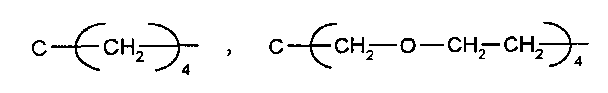 und

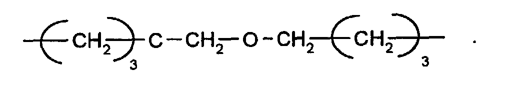

Vorzugsweise stehen R¹_{f} und R²_{f} unabhängig voneinander jeweils für einen Perfluoralkylrest mit 1 bis 6 C-Atomen oder bilden gemeinsam eine 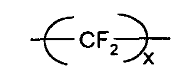 -Brücke, wobei x für eine ganze Zahl von 1 bis 5 steht.
- R³: steht vorzugsweise für Wasserstoff, Fluor, Chlor oder einen Perfluoralkylrest mit 1 bis 6 C-Atomen.

Besonders bevorzugt stehen R¹_{f} und R²_{f} unabhängig voneinander jeweils für Trifluormethyl oder Pentafluorethyl oder bilden gemeinsam eine 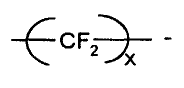 Brücke, wobei x für eine ganze Zahl von 2 bis 4 steht.
- R³: steht besonders bevorzugt für Fluor, Chlor, Trifluormethyl oder Pentafluorethyl.

Die vorliegende Erfindung betrifft auch ein Verfahren zur Herstellung von Fluoralkenylgruppen enthaltenden (Meth-)Acrylaten der Formel (I), das dadurch gekennzeichnet ist, daß man einen Alkohol der Formel (II) 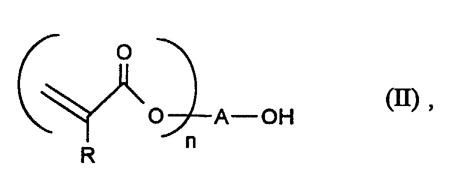 in der
R, n und A die bei Formel (I) angegebene Bedeutung haben,
in Gegenwart einer Base mit einem Olefin der Formel (III) umsetzt 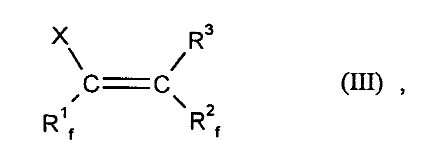 in der
- R¹_{f}, R²_{f} und R³: die bei Formel (I) angegebene Bedeutung haben und
- X: für Fluor oder Chlor steht.

In den Formeln (II) und (III) sind die bevorzugten und besonders bevorzugten Bedeutungen von n, A, R¹_{f} , R²_{f} und R³ ebenfalls so, wie bei Formel (I) angegeben.

Alkohole der Formel (II) sind meist bekannte Handelsprodukte oder analog zu bekannten Verbindungen herstellbar.
Ganz besonders bevorzugte Alkohole der Formel (II) sind Glycerindimethacrylat, Glycerinmethacrylatacrylat, Glycerindiacrylat, Pentaerythritoltriacrylat und Dipentaerythritolpentaacrylat.

Olefine der Formel (III) sind ebenfalls meist bekannte Handelsprodukte oder analog zu bekannten Verbindungen herstellbar.

Ganz besonders bevorzugte Olefine der Formel (III) sind Perfluor-(2-methylpenten-2), Hexafluorpropentrimer, 1,1,1,4,4,4-Hexafluor-2,3-dichlor-buten-2, Perfluorcyclobuten, Perfluorcyclopenten, Perfluorcyclohexen, Perfluorcyclopropen, 1,2-Dichlor-tetrafluorcyclobuten, 1,2-Dichlor-hexafluorcyclopenten und 1,2-Dichlor-octafluorcyclohexen.

Bezogen auf 1 Mol Alkohol der Formel (II) kann man beispielsweise 1 bis 20 Mol eines Olefins der Formel (III) einsetzen. Vorzugsweise beträgt diese Menge 1 bis 3 Mol.

Als Basen kommen anorganische und organische Basen infrage, beispielsweise Ammoniak, primäre, sekundäre und tertiäre Amine, Pyridine, Alkali- und Erdalkalihydroxide und Alkali- und Erdalkalicarbonate. Organische Amine können z.B. Alkylgruppen mit 1 bis 10 C-Atomen und/oder Arylgruppen mit 6 bis 10 C-Atomen enthalten. Bevorzugt sind Triethylamin, Dimethylanilin, Pyridin, Kaliumhydroxid, Natriumhydroxid, Kaliumcarbonat und Natriumcarbonat.

Die Menge der Base wird mindestens so gewählt, daß der bei der Umsetzung gebildete Fluor- oder Chlorwasserstoff gerade vollständig gebunden wird. Überschüsse an Base, z.B. bis zu 5 Äquivalenten stören im allgemeinen nicht.

Es ist vorteilhaft, das erfindungsgemäße Verfahren in Gegenwart eines Lösungsmittels durchzuführen. Geeignete Lösungsmittel sind aprotische Lösungsmittel wie Methylenchlorid, Chloroform, Tetrachlorkohlenstoff, Dichlorethan, Trichlorethan, Tetrachlorethan, Benzol, Toluol, Chlorbenzol, Dimethylformamid, Tetramethylensulfon, Dimethylsulfoxid, Acetonitril, Glyme, Diglyme und Tetrahydrofuran. Bezogen auf 100 g Alkohol der Formel (II) plus Olefin der Formel (III) kann man beispielsweise 40 bis 500 ml eines aprotischen Lösungsmittels einsetzen.

Die erfindungsgemäße Umsetzung kann man beispielsweise bei Temperaturen im Bereich 0 bis 80°C, vorzugsweise 10 bis 40°C, durchführen. Der Druck ist im allgemeinen nicht kritisch. Mann kann bei Normaldruck, Unterdruck und Überdruck arbeiten, beispielsweise bei 0,1 bis 5 bar. Vorzugsweise arbeitet man bei Normaldruck.

Die Reaktionszeit kann z.B. zwischen 0,5 und 15 Stunden betragen.

Man kann das erfindungsgemäße Verfahren beispielsweise so durchführen, daß man den Alkohol der Formel (II), das Olefin der Formel (III) und das Lösungsmittel vorlegt und portionsweise die Base zufügt. Die Zusammenführung der Reaktanden, der Base und gegebenenfalls des Lösungsmittels kann auch auf andere Weise erfolgen.

Das nach der Reaktion vorliegende Gemisch kann man beispielsweise so aufarbeiten, daß man es zunächst mit Wasser versetzt, die organische Phase abtrennt und daraus das evtl. vorhandene Lösungsmittel entfernt. Es ist vorteilhaft, die nach der Phasentrennung verbleibende wäßrige Phase mit einem mit Wasser nicht mischbaren Lösungsmittel zu extrahieren und das Extrakt zusammen mit der organischen Phase weiter aufzuarbeiten. Es ist weiterhin vorteilhaft, die organische Phase gegebenenfalls zusammen mit dem Extrakt aus der wäßrigen Phase vor der Entfernung von Lösungsmitteln zu trocknen, beispielsweise mit Natriumsulfat.

Bei bestimmten Lösungsmitteln kann beim Versetzen mit Wasser eine Phasentrennung ausbleiben. In solchen Fällen ist es zweckmäßig, das mit Wasser versetzte Reaktionsgemisch mit einem mit Wasser nicht mischbaren Lösungsmittel zu extrahieren und das Extrakt wie oben für die organische Phase angegeben aufzuarbeiten.

Nach dem erfindungsgemäßen Verfahren erhält man (Meth-)Acrylate der Formel (I) in hohen Ausbeuten von im allgemeinen über 80, häufig über 90 % d. Th.

Die Fluoralkenylgruppen enthaltenden (Meth-)Acrylate der Formel (I) können verwendet werden, um daraus Beschichtungen herzustellen.

Beispielsweise können dazu die (Meth-)Acrylate der Formel (I), gegebenenfalls im Gemisch mit anderen Monomeren und/oder Polymeren, zusammen mit einem Photoinitiator in einem inerten Lösungsmittel gelöst, mit einer Rakel auf das zu beschichtende Substrat aufgetragen und mit UV-Licht ausgehärtet werden. Als Photoinitiatoren kommen z.B. Benzoinderivate in Frage, als inerte Lösungsmittel z.B. Ketone wie Butanon und Ester. Wenn man Gemische von (Meth-)Acrylaten der Formel (I) mit anderen Monomeren und/oder Polymeren zur Herstellung der Beschichtungen einsetzt, dann kann deren Gehalt an (Meth-)Acrylaten der Formel (I) beispielsweise 2,5 bis 80 Gew.-% betragen. Vorzugsweise liegt diese Menge bei 5 bis 60 Gew.-%.

Die so hergestellten Beschichtungen zeichnen sich durch große Witterungsstabilität, große Hydrolysebeständigkeit aus und ausgeprägte Antihafteigenschaften aus.

### Beispiele

### Beispiel 1

Zu 150 g Glycerindimethacrylat und 200 g Perfluor-(2-Methyl-penten-2) in 500 ml Acetonitril wurden bei Raumtemperatur 100 g Triethylamin zugetropft. Nach 10 Stunden Rühren bei Raumtemperatur wurden 500 ml Wasser zugefügt und die organische Phase abgetrennt. Die wäßrige Phase wurde zweimal mit 200 ml Methylenchlorid extrahiert. Die vereinigten organischen Phasen wurden nacheinander mit 100 ml Wasser, 100 ml 1 N wäßriger Salzsäure und 100 ml Wasser extrahiert. Die organische Phase wurde anschließend über Natriumsulfat getrocknet. Nach Entfernung des Lösungsmittels im Vakuum wurden 270 g 2-Methyl-acrylsäure-[3-(2-Methyl-acryloyloxy)-2-(3,3,3-trifluor-1-(1,1,2,2,2-pentafluorethyl)-2-trifluormethyl-propenyloxy)]-propylester erhalten.
Ausbeute: 82 % d.Th.
¹⁹F-NMR-Daten: δ (CDCl₃) -56,2, -59,6, -80,6, -113,4 ppm
¹H-NMR-Daten: δ (CDCl₃) 1,95 (s, 6H), 4,4 (m, 4H), 5,0 (m,1H), 5,65 (m, 2H), 6,15 (m, 2H) ppm.

### Beispiel 2

Zu 11,4 g Glycerindimethacrylat und 10,6 g Perfluorcyclopenten in 30 ml Acetonitril wurden bei Raumtemperatur 10 g Triethylamin zugetropft. Nach 10 Stunden Rühren bei Raumtemperatur wurden 100 ml Wasser zugefügt und die organische Phase abgetrennt. Die wäßrige Phase wurde zweimal mit je 50 ml Methylenchlorid extrahiert. Die vereinigten organischen Phasen wurden nacheinander mit 50 ml Wasser, 50 ml 1N wäßriger Salzsäure und 50 ml Wasser extrahiert. Die vereinigten organischen Phasen wurden anschließend über Natriumsulfat getrocknet. Nach Entfernung des Lösungmittels im Vakuum wurden 19 g 2-Methyl-acrylsäure-[3-(2-Methyl-acryloyloxy)-2-(2,3,3,4,4,5,5-heptafluorcyclopentenyloxy)]-propylester erhalten.
Ausbeute: 90 % d. Th.
¹⁹F-NMR-Daten: δ (CDCl₃)-115,6, -116,5, -130,1, -159 ppm
¹H-NMR-Daten: δ (CDCl₃) 1,95 (s,6H), 4,4 (m,4H), 5,05 (m,1H), 5,65 (m,2H), 6,15 (m,2H) ppm.

### Beispiel 3

Zu 15 g Glycerin-methacrylat-acrylat und 27 g Perfluor-(2-Methyl-penten-2) in 100 ml Acetonitril wurden bei Raumtemperatur 15 g Triethylamin zugetropft. Nach 3 Stunden Rühren bei Raumtemperatur wurden 300 ml Wasser zugefügt und die organische Phase abgetrennt. Die wäßrige Phase wurde zweimal mit je 150 ml Methylenchlorid extrahiert. Die vereinigten organischen Phasen wurden nacheinander mit 100 ml Wasser, 100 ml 1N wäßriger Salzsäure und 100 ml Wasser extrahiert. Die vereinigten organischen Phasen wurden anschließend über Natriumsulfat getrocknet. Nach Entfernung des Lösungsmittels im Vakuum wurden 33 g 2-Methyl-acrylsäure-[3-acryloyloxy-2-(3,3,3-trifluor-1-(1,1,2,2,2-pentafluorethyl)-2-trifluormethyl-propenyloxy)]-propylester erhalten.
Ausbeute: 96 % d. Th.
¹⁹F-NMR-Daten: δ (CDCl₃) -56,3, -59,6, -80,6, -113,4 ppm
¹H-NMR-Daten: δ (CDCl₃) 1,95 (s, 3H), 4,4 (m, 4H), 5,0, 5,4 (m, 1H), 5,65 (m, 1H), 5,95 (m, 1H), 6,15 (m, 2H), 6,45 (m, 1H) ppm.

### Beispiel 4

Zu 21 g Glycerin-methacrylat-acrylat und 21 g Perfluorcyclopenten in 100 ml Acetonitril wurden bei Raumtemperatur 20 g Triethylamin zugetropft. Nach 6 Stunden Rühren bei Raumtemperatur wurden 100 ml Wasser zugefügt und die organische Phase abgetrennt. Die wäßrige Phase wurde zweimal mit je 50 ml Methylenchlorid extrahiert. Die vereinigten organischen Phasen wurden nacheinander mit 50 ml Wasser, 50 ml 1N wäßriger Salzsäure und 50 ml Wasser extrahiert. Die vereinigten organischen Phasen wurden anschließend über Natriumsulfat getrocknet. Nach Entfernung des Lösungsmittels im Vakuum wurden 37 g 2-Methylacrylsäure-[3-acryloyloxy-2-(2,3,3,4,4,5,5-heptafluorcyclopentenyloxy)]-propylester erhalten.
Ausbeute: 93 % d. Th.
¹⁹F-NMR-Daten: δ (CDCl₃) -115,4, -116,4, -130,0, -158,9 ppm
¹H-NMR-Daten: δ (CDCl₃) 1,95 (s, 3H), 4,4 (m, 4H), 5,1, 5,48 (m, 1H), 5,65 (m, 1H), 5,95 (m, 1H), 6,15 (m, 2H), 6,45 (m, 1H) ppm.

### Beispiel 5

Aus den unten angegebenen Monomeren wurden unter Zusatz von jeweils 0,5 g Benzyldimethylketal (= Photoinitiator) und jeweils 79,5 g Butanon (= Lösungsmittel) Lösungen hergestellt:
a) 20 g Produkt aus Beispiel 1,
b) 10 g Produkt aus Beispiel 1 und 10 g BGMA,
c) 10 g Produkt aus Beispiel 1 und 10 g UMA,
d) 5 g Produkt aus Beispiel 1 und 15 g BGMA,
e) 20 g Produkt aus Beispiel 2,
f) 10 g Produkt aus Beispiel 2 und 10 g BGMA,
g) 20 g Produkt aus Beispiel 3 und
h) 20 g Produkt aus Beispiel 3 und 10 g BGMA.

- BGMA =: Bisphenol-A-diglycidyldimethacrylat.
- UMA =: Umsetzungsprodukt aus einem Mol 2,2,4-Trimethylhexamethylendiisocyanat und 2 Molen 2-Hydroxyethylmethacrylat.

Die Lösungen a) bis h) wurden getrennt voneinander mit einer Rakel auf eine Polyethylenterephthalat-Folie so aufgebracht, daß sich Naßfilmdicken von jeweils 100 µm ergaben. Diese Schichten wurden bei 40°C 30 Minuten lang getrocknet und anschließend in einer UV-Belichtungseinheit (2000 Watt) unter Luftausschluß belichtet. Es wurden jeweils Beschichtungen erhalten, die klebefrei, hart, wasserabweisend und schmutzabweisend waren und sich durch besondere Alterungsstabilität und Hydrolysefestigkeit auszeichneten.

## Patentansprüche

1. Fluoralkenylgruppen enthaltende (Meth-)Acrylate der Formel (I) 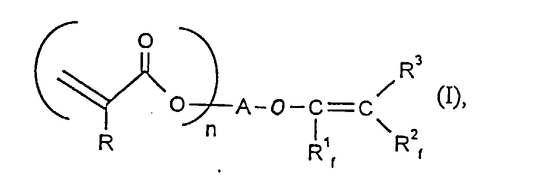 in der
R für Wasserstoff oder Methyl,
n für eine ganze Zahl von 2 bis 6,
A für einen (n+1)-wertigen, gegebenenfalls bis zu 10 Sauerstoffatome enthaltenden Kohlenwasserstoffrest mit 3 bis 30 C-Atomen und
R¹_{f} und R²_{f} unabhängig voneinander jeweils für einen Perfluoralkylrest mit 1 bis 20 C-Atomen stehen oder
R¹_{f} und R²ᵣ gemeinsam eine 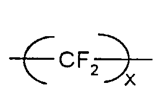 -Brücke bilden, wobei
x für eine ganze Zahl von 1 bis 8 steht und
R³ für Wasserstoff, Fluor, Chlor oder einen Perfluoralkylrest mit 1 bis 20 C-Atomen steht.

2. Fluoralkenylgruppen enthaltende (Meth-)Acrylate des Anspruchs 1, dadurch gekennzeichnet, daß in Formel (I)
R für Wasserstoff oder Methyl,
n für eine ganze Zahl von 2 bis 5,
A für einen (n + 1)-wertigen, gegebenenfalls bis zu 6 OH-Gruppen und/oder gegebenenfalls bis zu 8 Etherbrücken enthaltenden Kohlenwasserstoffrest mit 3 bis 12 C-Atomen und
R¹_{f} und R²_{f} unabhängig voneinander jeweils für einen Perfluoralkylrest mit 1 bis 6 C-Atomen stehen oder
R¹_{f} und R²_{f} gemeinsam eine 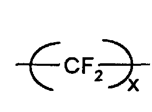 -Brücke bilden, wobei
x für eine ganze Zahl von 1 bis 5 steht und
R³ für Fluor, Chlor oder einen Perfluoralkylrest mit 1 bis 6 C-Atomen steht.

3. Verfahren zur Herstellung von Fluoralkenylgruppen enthaltenden (Meth-)-Acrylaten des Anspruchs 1, dadurch gekennzeichnet, daß man einen Alkohol der Formel (II) 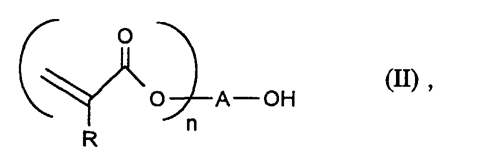 in der
R, n und A die in Anspruch 1 angegebene Bedeutung haben,
in Gegenwart einer Base mit einem Olefin der Formel (III) umsetzt 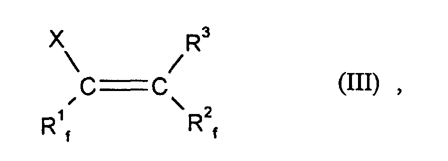 in der
R¹_{f}, R²_{f} und R³ die in Anspruch 1 angegebene Bedeutung haben und
X für Fluor oder Chlor steht.

4. Verfahren nach Anspruch 3, dadurch gekennzeichnet, daß man als Alkohole der Formel (II) Glycerindimethacrylat, Glycerinmethacrylatacrylat, Glycerindiacrylat, Pentaerythritoltriacrylat oder Dipentaerythritolpentaacrylat und als Olefine der Formel (III) Perfluor-(2-Methyl-penten-2), Hexafluorpropen-Trimer, 1,1,1,4,4,4-Hexafluor-2,3-dichlor-buten-2, Perfluorcyclobuten, Perfluorcyclopenten, Perfluorcyclohexen, Perfluorcydopropen, 1,2-Dichlor-tetrafluorcyclobuten, 1,2-Dichlor-hexafluorcyclopenten oder 1,2-Dichlor-octafluorcyclohexen einsetzt.

5. Verfahren nach Ansprüchen 3 bis 4, dadurch gekennzeichnet, daß man auf 1 Mol Alkohol der Formel (II) 1 bis 20 Mol eines Olefins der Formel (III) einsetzt.

6. Verfahren nach Ansprüchen 3 bis 5, dadurch gekennzeichnet, daß man als Base mindestens eine äquivalente Menge Ammoniak, primäre, sekundäre oder tertiäre Amine, Pyridine, Alkali- oder Erdalkalihydroxide oder Alkali-oder Erdalkalicarbonate einsetzt.

7. Verfahren nach Ansprüchen 3 bis 6, dadurch gekennzeichnet, daß man in Gegenwart eines Lösungsmittels arbeitet.

8. Verfahren nach Ansprüchen 3 bis 7, dadurch gekennzeichnet, daß man bei einer Temperatur im Bereich 0 bis 80°C arbeitet.

9. Verwendung von Fluoralkenylgruppen enthaltenden (Meth-)Acrylaten des Anspruchs 1 zur Herstellung von Beschichtungen.

## Claims

1. (Meth)acrylates containing fluoroalkenyl groups, of the formula (I) 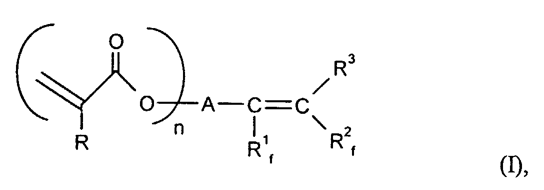 in which
R represents hydrogen or methyl,
n represents an integer from 2 to 6,
A represents a (n+1)-valent hydrocarbon radical having 3 to 30 carbon atoms which optionally contains up to 10 oxygen atoms, and
R¹_{f} and R²_{f} independently of one another each represent a perfluoroalkyl radical having 1 to 20 carbon atoms, or
R¹_{f} and R²_{f} together form a 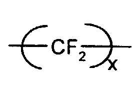 bridge, in which
x represents an integer from 1 to 8, and
R³ represents hydrogen, fluorine, chlorine or a perfluoroalkyl radical having 1 to 20 carbon atoms.

2. (Meth)acrylates containing fluoroalkenyl groups, of Claim 1, characterized in that in formula (I)
R represents hydrogen or methyl,
n represents an integer from 2 to 5,
A represents a (n+1)-valent hydrocarbon radical having 3 to 12 carbon atoms which optionally contains up to 6 OH groups and/or optionally up to 8 ether bridges, and
R¹_{f} and R²_{f} independently of one another each represent a perfluoroalkyl radical having 1 to 6 carbon atoms, or
R¹_{f} and R²_{f} together form a 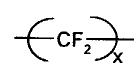 bridge in which
x represents an integer from 1 to 5, and
R³ represents fluorine, chlorine or a perfluoroalkyl radical having 1 to 6 carbon atoms.

3. Process for the preparation of (meth)acrylates containing fluoroalkenyl groups, of Claim 1, characterized in that an alcohol of the formula (II) 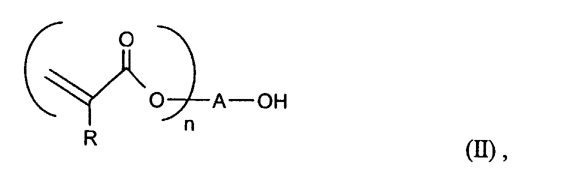 in which
R, n and A have the meaning given in Claim 1,
is reacted in the presence of a base with an olefin of the formula (III) 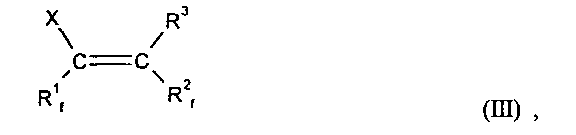 in which
R¹_{f}, R²_{f} and R³ have the meaning given in Claim 1 and
X represents fluorine or chlorine.

4. Process according to Claim 3, characterized in that alcohols of the formula (II) employed are glycerol dimethacrylate, glycerol methacrylate acrylate, glycerol diacrylate, pentaerythritol triacrylate or dipentaerythritol pentaacrylate and olefins of the formula (III) employed are perfluoro-(2-methyl-pent-2-ene), hexafluoropropene trimer, 1,1,1 ,4,4,4-hexafluoro-2,3-dichloro-but-2-ene, perfluorocyclobutene, perfluorocyclopentene, perfluorocyclohexene, perfluorocyclopropene, 1,2-dichloro-tetrafluorocyclobutene, 1,2-dichlorohexafluorocyclopentene or 1,2-dichloro-octafluorocyclohexene.

5. Process according to Claims 3 to 4, characterized in that from 1 to 20 mol of an olefin of the formula (III) are employed per mole of alcohol of the formula (II).

6. Process according to Claims 3 to 5, characterized in that at least one equivalent quantity of ammonia, primary, secondary or tertiary amines, pyridines, alkali metal or alkaline earth metal hydroxides or alkali metal or alkaline earth metal carbonates is employed as base.

7. Process according to Claims 3 to 6, characterized in that it is carried out in the presence of a solvent.

8. Process according to Claims 3 to 7, characterized in that it is carried out at a temperature in the range from 0 to 80°C,

9. Use of (meth)acrylates containing fluoroalkenyl groups, of Claim 1, for the preparation of coatings.

## Revendications

1. (Méth)acrylates contenant des groupes fluoroalcényle de formule (I) 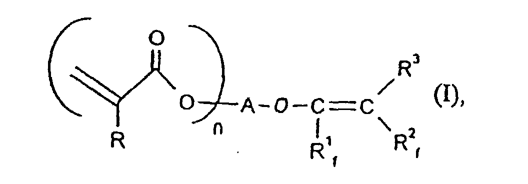 dans laquelle
R représente un atome d'hydrogène ou un groupe méthyle,
n représente un nombre entier de 2 à 6,
A représente un reste hydrocarboné de valence (n+1) de 3 à 30 atomes de carbone, contenant éventuellement jusqu'à 10 atomes d'oxygène, et
R¹_{f} et R²_{f} représentent chacun, indépendamment l'un de l'autre, un reste perfluoroalkyle de 1 à 20 atomes de carbone, ou
R¹_{f} et R²_{f} forment ensemble un pont 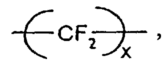 où x représente un nombre entier de 1 à 8 et et
R³ représente un atome d'hydrogène, de fluor ou de chlore ou un reste perfluoroalkyle de 1 à 20 atomes de carbone.

2. (Méth)acrylates contenant des groupes fluoroalcényle selon la revendication 1, caractérisés en ce que, dans la formule (I):
R représente un atome d'hydrogène ou un groupe méthyle,
n représente un nombre entier de 2 à 5,
A représente un reste hydrocarboné de valence (n+1) de 3 à 12 atomes de carbone, contenant éventuellement jusqu'à 6 groupes OH et/ou éventuellement jusqu'à 8 ponts éther, et
R¹_{f} et R²_{f} représentent chacun, indépendamment l'un de l'autre, un reste perfluoroalkyle de 1 à 6 atomes de carbone, ou
R¹_{f} et R²_{f} forment ensemble un pont 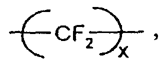 où x représente un nombre entier de 1 à 5 et
R³ représente un atome de fluor ou de chlore ou un reste perfluoroalkyle de 1 à 6 atomes de carbone.

3. Procédé de préparation de (méth)acrylates contenant des groupes fluoroalcényle selon la revendication 1, caractérisé en ce que l'on fait réagir un alcool de formule (II) 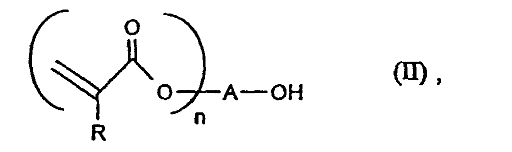 dans laquelle R, n et A ont la signification donnée dans la revendication 1,
en présence d'une base, avec une oléfine de formule (III) 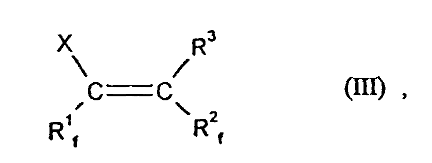 dans laquelle R¹_{f}, R²_{f} et R³ ont la signification donnée dans la revendication 1, et X représente le fluor ou le chlore.

4. Procédé selon la revendication 3, caractérisé en ce que l'on utilise comme alcools de formule (II) le diméthacrylate de glycérol, le méthacrylate et acrylate de glycérol, le diacrylate de glycérol, le triacrylate de pentaérythritol ou le pentacrylate de dipentaérythritol, et comme oléfines de formule (III) le perfluoro(2-méthylpenténe-2), le trimère de l'hexafluoropropène, le 1,1,1,4,4,4-hexafluoro-2,3-dichlorobutène-2, le perfluorocylobutène, le perfluorocydopentène, le perfluorocyclohexène, le perfluorocydopropène, le 1,2-dichlorotétrafluorocydobutène, le 1,2-dichlorohexafluorocyclopentène ou le 1 ,2-dichloro-octafluorocyclohexène.

5. Procédé selon les revendications 3 à 4, caractérisé en ce que l'on utilise 1 à 20 mol d'oléfine de formule (III) pour 1 mol d'alcool de formule (II).

6. Procédé selon les revendications 3 à 5, caractérisé en ce que l'on utilise comme base au moins une quantité équivalente d'ammoniac, d'amines primaires, secondaires ou tertiaires, de pyridines, d'hydroxydes de métaux alcalins ou de métaux alcalino-terreux ou de carbonates de métaux alcalins ou de métaux alcalino-terreux.

7. Procédé selon les revendications 3 à 6, caractérisé en ce que l'on travaille en présence d'un solvant.

8. Procédé selon les revendications 3 à 7, caractérisé en ce que l'on travaille à une température comprise entre 0 et 80°C.

9. Utilisation de (méth)acrylates contenant des groupes fluoroalcényle selon la revendication 1 pour la préparation de revêtements.
